(19) Europäisches Patentamt

European Patent Office

Office européen des brevets

(11) **EP 0 590 241 B1**

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(51) Int. Cl.⁶: **A61F 2/40**

(21) Anmeldenummer: 93109828.9

(22) Anmeldetag: **19.06.1993**

(54) **Künstliches Schultergelenk**

Artificial shoulder joint

Articulation artificielle de l'épaule

(84) Benannte Vertragsstaaten:
**AT BE CH DE DK ES FR GB GR IE IT LI LU MC NL PT SE**

(30) Priorität: **26.09.1992 DE 4232313**

(43) Veröffentlichungstag der Anmeldung:
**06.04.1994 Patentblatt 1994/14**

(73) Patentinhaber:
• **Kubein-Meesenburg, Dietmar, Prof. Dr.**
**37547 Kreiensen (DE)**
• **Theusner, Joachim, Dr.**
**80539 München (DE)**
• **NÄGERL, Hans Dr.**
**37130 Gleichen (DE)**

(72) Erfinder:
• **Kubein-Meesenburg, Dietmar, Prof., Dr.**
**D-3350 Kreiensen 1 (DE)**
• **Nägerl, Hans, Dr.**
**D-3407 Gleichen/OT, Klein-Lengden (DE)**

(74) Vertreter: **Patentanwälte**
**Dr. Solf & Zapf**
**Postfach 13 01 13**
**42028 Wuppertal (DE)**

(56) Entgegenhaltungen:
EP-A- 0 360 734          WO-A-90/11062
FR-A- 2 541 890          US-A- 4 261 062
US-A- 4 550 450          US-A- 4 964 865
US-A- 5 032 132

# Beschreibung

Die vorliegende Erfindung betrifft eine Endoprothese für das menschliche Schultergelenk.

Allgemein wird bisher davon ausgegangen, daß das Schultergelenk zu den einfachen Gelenkkonstruktionen gehört und als Kugelgelenk angesehen werden kann, wobei diesem Kugelgelenk drei Rotationsfreiheitsgrade zugebilligt werden.

Weiterhin ist es bereits aus der WO-A-90/11062 entsprechend der deutschen Patentanmeldung P 39 08 958.4 bekannt, daß künstliche Gelenke zum Einsatz im menschlichen Körper mit zwei Gelenkteilen mit zueinander sich bewegenden sphärischen Funktionsflächen derartige Krümmungsverhältnisse der Funktionsflächen besitzen, und zwar bei einer beispielsweise konvex-konkaven Konfiguration der Funktionsflächen, daß eine Gelenkgeometrie vorhanden ist, die durch eine Gelenkkette mit zwei Gelenkachsen bestimmt ist, und zwar einer sogenannten dimeren Gelenkkette. Hierbei können sich die Rotationszentren der beiden Funktionsflächen innerhalb des Gelenkteiles mit der konvexen Funktionsfläche befinden, so daß sich eine stabile Gelenkkette ergibt. Voraussetzung für die Funktion dieser dimeren Gelenkketten ist ein vorhandener Kraftschluß im Berührungspunkt der beiden Funktionsflächen.

Der vorliegenden Erfindung liegt die Aufgabe zugrunde, die bisher bekannten Endoprothesen für das menschliche Schultergelenk, die sämtlich auf dem Prinzip des Kugelgelenkes aufgebaut sind, unter Anwendung der Erkenntnis der Funktionsweise der dimeren Gelenkkette dahingehend zu verbessern, daß eine Endoprothese für das menschliche Schultergelenk geschaffen wird, die weitestgehend die natürliche Gelenkfunktion ermöglicht.

Erfindungsgemäß wird dies mit einem Schultergelenk erreicht, bestehend aus zwei zueinander sich bewegenden Gelenkteilen, einem Gelenkkopf und einer Gelenkpfanne, mit kugelförmigen Gelenkflächen, wobei die Krümmungsverhältnisse ihrer Funktionsflächen sowohl in der Frontalebene (Vertikalebene) als auch in einer hierzu senkrechten Querebene konvex-konkav sind, und die Gelenkgeometrie der Funktionsflächen zueinander in jeder der beiden Ebenen durch eine dimere Gelenkkette d.h. einer Gelenkkette mit zwei Gelenkachsen bestimmt ist, die durch die Rotationszentren der Funktionsflächen der jeweiligen Schnittkonturen verläuft, wobei der Gelenkkopf und die Gelenkpfanne in bezug auf ihre Funktionsflächen derart ausgestaltet sind, daß in ihrer der Ruhestellung des Armes entsprechenden Lage im hängenden Armzustand die beiderseitigen Funktionsflächen in der Frontalebene sich linienförmig berühren und denselben Krümmungsradius $R_{K1} = R_{P1}$ der Funktionsflächen besitzen, und hieran sich ein Abrollbereich anschließt, in dem die Radien der beiden Funktionsflächen derart gewählt sind, daß sich zwischen den beiden Gelenkteilen in Umfangsrichtung ein sich stetig erweiternder Spaltabschnitt ergibt. Weiterhin ist erfindungsgemäß vorgesehen, in der Querebene, die in radialer Richtung durch den jeweiligen Berührungspunkt der Funktionsfläche der Gelenkteile verläuft, die Radien der Funktionsflächen derart zu bemessen, daß sich jeweils beidseitig des Berührungspunktes der Funktionsflächen ein sich umfangsgemäß erweiternder Spalt erstreckt. Das erfindungsgemäße Gelenk gewährleistet fünf Freiheitsgrade. Die erfindungsgemäße Gelenkgestaltung basiert auf der Erkenntnis, daß der Gelenkkopf sich einerseits um seinen eigenen Krümmungsmittelpunkt mit einer bestimmten Winkelgeschwindigkeit dreht, wobei der Kontaktpunkt auf der Gelenkfläche der Gelenkpfanne an Ort und Stelle verbleibt, und der Kontaktpunkt auf dem, Gelenkkopf im Gegensinn zur Rotation wandert. Zusätzlich dreht sich der Gelenkkopf um den Mittelpunkt der Gelenkpfanne mit einer bestimmten Winkelgeschwindigkeit, wobei der Kontaktpunkt auf dem Gelenkkopf liegen bleibt, aber auf der Funktionsfläche der Gelenkpfanne im gleichen Drehsinn wandert. Diese beiden vorstehenden Rotationen überlagern sich. Hierbei gewährleistet weiterhin die vorliegende Erfindung, daß in der Ruhestellung die Gelenkpfanne in ihrem unteren Teil belastet wird, so daß eine linienförmige Auflage der Funktionsflächen vorhanden ist, wodurch eine optimale Krafteinleitung erreicht wird. Weiterhin wird durch die erfindungsgemäße Ausbildung des Gelenkspaltes erreicht, der durch die Inkongruenz der Krümmungsradien der artikulierenden Funktionsflächen gegeben ist, daß bei einem Anheben des Armes der Kontaktpunkt der Funktionsfläche nach oben wandert, so daß der Gelenkspalt nach oben hin kleiner wird und sich nach unten hin vergrößert. Dies führt zu einer Druckerhöhung der Gelenkflüssigkeit im oberen Gelenkspalt und im unteren Bereich zu einer Druckabsenkung. Hierzu kommt noch, daß jener Punkt der Gelenkfläche des Gelenkkopfes, der momentan den Kontaktpunkt bildet, trotz der Kontaktpunktwanderung nach oben eine Geschwindigkeit V besitzt, die nach unten gerichtet ist, und die Gelenkflüssigkeit nach unten "mitnehmen" kann. Somit arbeitet das erfindungsgemäße Gelenk nach dem Prinzip einer Kreiselpumpe.

Weitere vorteilhafte Ausführungen sind in den Unteransprüchen enthalten.

Anhand des in den beiliegenden Zeichnungen dargestellten Ausführungsbeispieles wird die Erfindung näher erläutert.

Es zeigen:

Fig. 1  einen Frontalschnitt durch ein erfindungsgemäßes Gelenk,

Fig. 2  einen Querschnitt zu der Schnittdarstellung in Fig. 1 in radialer Richtung durch den Berührungspunkt der Funktionsflächen der Gelenkteile,

Fig. 3  einen Frontalschnitt durch ein erfindungsgemäßes Gelenk in einer angehobenen Stellung des Armes.

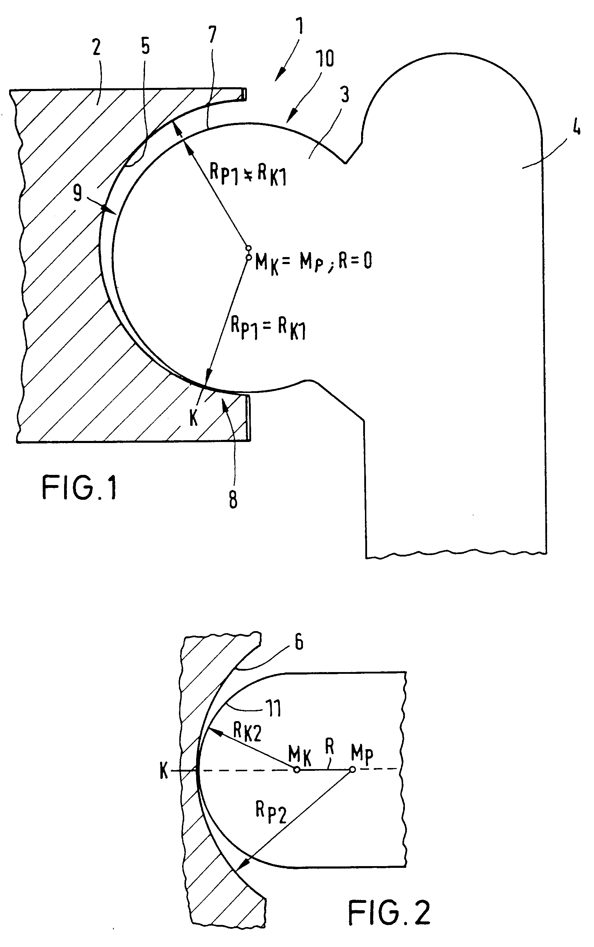

In der Fig. 1 ist ein erfindungsgemäßes künstliches Gelenk 1 zum Ersatz des menschlichen Schultergelenkes dargestellt. Dieses künstliche Gelenk 1 besteht aus einer Gelenkpfanne 2 und einem Gelenkkopf 3. Hierbei ist der Gelenkkopf 3 mit dem Oberarmknochen 4 verbunden. Die Gelenkpfanne 2 hat eine konkave Krümmung mit einem Krümmungsradius $R_{P1}$ in der Frontalebene, wobei die hierdurch gebildete Funktionsfläche 5 der Gelenkpfanne kreisförmig ist, das heißt der Krümmungsradius $R_{P1}$ ist über den gesamten Funktionsflächenbereich konstant. Er beträgt zweckmäßigerweise ca. 25 mm. Aus Fig. 2 ist zu erkennen, daß auch in der zur Frontalebene gemäß Fig. 1 senkrechten Querebene die Gelenkpfanne 2 eine Funktionsfläche 6 besitzt, die einen konstanten Krümmungsradius $R_{P2}$ besitzt, und zwar über den gesamten Bereich der Funktionsfläche. Hierbei ist zusätzlich vorgesehen, daß beide Krümmungsradien der Funktionsflächen 5 und 6 gleich groß sind, das heißt $R_{P1} = R_{P2}$. Der Gelenkkopf 3 besitzt eine sphärische Oberfläche, wobei der Krümmungsradius $R_{K1}$ der Funktionsfläche 7 in der frontalen Schnittebene nicht konstant ist. Vielmehr ist der Krümmungsradius RK1 derart bemessen, daß in der in Fig. 1 dargestellten Ruhestellung des erfindungsgemäßen Gelenkes, die der Stellung des menschlichen Gelenkes bei einem hängenden Arm entspricht, im unteren Bereich der Gelenkpfanne 2 der Gelenkkopf 3 innerhalb eines Auflagebereichs 8 einen Krümmungsradius besitzt, der im Krümmungsradius $R_{P1}$ der Gelenkpfanne 2 entspricht. Hierdurch wird in diesem Auflagebereich 8 eine linienförmige Berührung zwischen den beiden Funktionsflächen 5, 7 von Gelenkkopf 3 und Gelenkpfanne 2 bewirkt. Dieser linienförmige Auflagebereich 8 kann etwa 20 ° umfangsgemäß am Gelenkkopf 3 betragen. An diesen linienförmigen Auflagebereich 8 schließt sich ein Abrollbereich 9 an, in dem die Radien der beiden Funktionsflächen 5, 7 von Gelenkpfanne 2 und Gelenkkopf 3 derart ausgestaltet sind, daß sich zwischen den beiden Gelenkteilen 2, 3 in Umfangsrichtung ein sich stetig erweiternder Spaltabschnitt ergibt. Dieser erweiternde Spaltabschnitt wird im gezeigten Ausführungsbeispiel dadurch erreicht, daß sich der Radius der Funktionsfläche 7 des Gelenkkopfes 3 - ausgehend von dem Radius im Auflagebereich 8 - stetig verringert. Diese Verringerung des Radius beträgt zweckmäßigerweise maximal 20 % des Radius der Funktionsflächen von dem Gelenkkopf 3 und der Gelenkpfanne 2 im Auflagebereich 8. Der Abrollbereich 9 erstreckt sich zweckmäßigerweise über einen Umfangsbereich des Gelenkkopfes von ca. 180 °. Weiterhin ist erfindungsgemäß vorgesehen, daß sich an den Abrollbereich 9 in Umfangsrichtung ein Endbereich 10 mit konstanter Radiendifferenz der Funktionsflächen von Gelenkkopf 3 und Gelenkpfanne 2 anschließt, der etwa 40 ° bis 70 ° des Umfanges des Gelenkkopfes 3 beträgt. In Fig. 2 ist zu erkennen, daß in der Querebene, die in radialer Richtung durch den jeweiligen Berührungspunkt K der Funktionsflächen der Gelenkteile 2, 3 verläuft, die Radien der Funktionsflächen 6, 11 derart bemessen sind, daß sich jeweils beidseitig des Berührungspunktes K ein sich umfangsgemäß erweiternder Spalt ergibt. Hierbei besitzt der Radius $R_{P2}$ der Funktionsfläche 6 der Gelenkpfanne 2 denselben Radius wie deren Funkktionsfläche 5 in der frontalen Ebene. Jedoch ist der Radius $R_{K2}$ der Funktionsfläche 11 des Gelenkkopfes 3 unterschiedlich von dem Radius der Funktionsfläche 7 des Gelenkkopfes in der frontalen Ebene ausgebildet. Hierbei ist es zweckmäßig, wenn die Radiendifferenz der beiden Funktionsflächen 6, 11 beidseitig des Berührungspunktes K maximal 30 % des Radius der Funktionsflächen im Auflagebereich 8 beträgt. Hierbei ist weiterhin zu erkennen, daß der Radius $R_{K2}$ des Gelenkkopfes 3 ca. um 12 % kleiner ist als der Radius der radiengleichen Funktionsflächen im Auflagebereich 8. Zweckmäßigerweise beträgt der Radius $R_{K2}$ ca. 22 mm. Hierbei reduziert sich der Radius $R_{K2}$ der Funktionsfläche des Gelenkkopfes in der Querebene von dem maximalen Wert im Berührungspunkt K in dorsaler Richtung um ca. 3 mm über ein Umfangswinkel von etwa 90 ° und in ventraler Richtung ebenfalls über einen Umfangswinkel von 90 ° um maximal 1 mm.

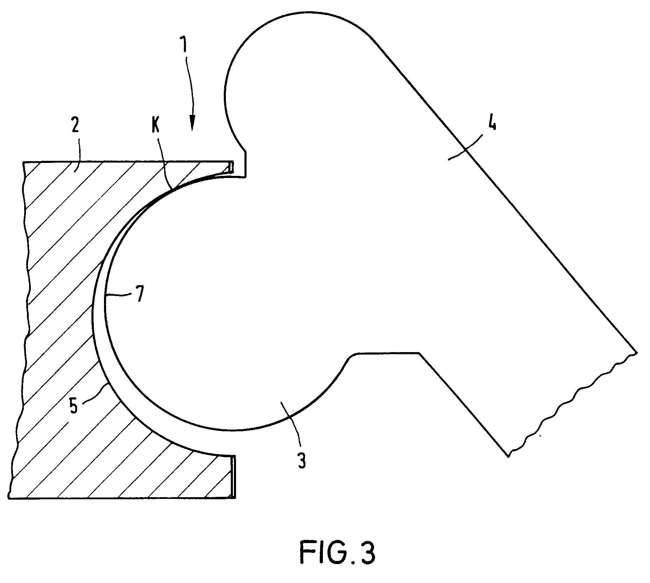

Wie sich aus der Lage der Mittelpunkte $M_K$ und $M_P$ der Funktionsflächen sowohl in der frontalen Ebene als auch in der Querebene ergibt, handelt es sich im vorliegenden Beispiel jeweils um sich zueinander bewegende Gelenkteile 2, 3 mit einer konvex-konkaven Ausbildung der Funktionsflächen 5, 7 bzw. 6, 11, wobei die Mittelpunkte der Funktionsflächen $M_K$ und $M_P$ jeweils in dem Gelenkteil mit der konvexen Funktionsfläche 7, 11, nämlich dem Gelenkkopf 3, liegen, und wobei die Bedingung erfüllt ist, daß sich eine dimere, stabile Gelenkkette ausbildet, wobei das Kettenglied R eine Gelenkachsenbahn mit dem Radius $R = R_P - R_K$ besitzt, wobei dieser Wert größer Null bzw. im Bereich der linienförmigen Auflage gleich Null ist. In Fig. 3 ist die Stellung eines erfindungsgemäßen Gelenkes 1 dargestellt bei einer um 60 ° angehobenen Armstellung.

Im Vorstehenden ist vorgesehen, daß die Gelenkpfanne in beiden Schnittebenen konstante und gleiche Radien der Funktionsflächen besitzt sowie die Radien der Funktionsflächen des Gelenkkopfes variieren. Es liegt aber ebenfalls im Rahmen der Erfindung, wenn die Radien der Funktionsflächen des Gelenkkopfes konstant und gleich sind und die Radien der Funktionsflächen der Gelenkpfanne variieren, so daß sich die erfindungsgemäßen Geometrien der Funktionsflächen ergeben.

**Patentansprüche**

1. Endoprothese für das menschliche Schultergelenk, bestehend aus zwei zueinander sich bewegenden Gelenkteilen, einem Gelenkkopf und einer Gelenkpfanne, mit kugelförmigen Gelenkflächen, wobei die Krümmungsverhältnisse ihrer Funktionsflächen sowohl in der Frontalebene als auch in einer hierzu senkrechten Querebene konvex-konkav sind, und die Gelenkgeometrie der Funktionsflächen zueinander in jeder der beiden Ebenen durch eine dimere Gelenkkette d.h. einer gelenkkette mit zwei gelenkachsen bestimmt ist, die durch die Rotationszentren der Funktionsflächen der jeweiligen Schnittkonturen verläuft,
**dadurch gekennzeichnet,** daß
der Gelenkkopf (3) und die Gelenkpfanne (2) in bezug auf ihre Funktionsflächen derart ausgestaltet sind, daß in ihrer der Ruhestellung des Armes entsprechenden Lage im hängenden Zustand die beiderseitigen Funktionsflächen (5, 7) in der Frontalebene sich linienförmig berühren und denselben Krümmungsradius im Auflagebereich (8) besitzen und hieran sich ein Abrollbereich (9) anschließt, in dem die Radien $R_{P1}$, $R_{K1}$ der beiden Funktionsflächen (5, 7) derart gewählt sind, daß sich zwischen den beiden Gelenkteilen (2, 3) in Umfangsrichtung ein sich erweiternder Spaltabschnitt erstreckt.

2. Künstliches Gelenk nach Anspruch 1,
**dadurch gekennzeichnet,** daß
in der Querebene, die in radialer Richtung durch den jeweiligen Berührungspunkt K der Funktionsflächen (6, 11) der Gelenkteile (2, 3) verläuft, die Radien $R_{P2}$, $R_{K2}$ der Funktionsflächen (6, 11) derart bemessen sind, daß sich jeweils beidseitig des Berührungspunktes K ein sich umfangsgemäß erweiternder Spalt ergibt.

3. Künstliches Gelenk nach Anspruch 1 oder 2,
**dadurch gekennzeichnet,** daß
der Abrollbereich (9) sich über einen Umfangsbereich des Gelenkkopfes (3) von ca. 180 ° erstreckt.

4. Künstliches Gelenk nach einem der Ansprüche 1 bis 3,
**dadurch gekennzeichnet,** daß
der Auflagebereich (8) sich über einen Umfangsbereich des Gelenkkopfes (3) von ca. 20 ° erstreckt.

5. Künstliches Gelenk nach einem der Ansprüche 1 bis 4,
**dadurch gekennzeichnet,** daß
sich an den Abrollbereich (9) in Umfangsrichtung ein Endbereich (10) mit konstanter Radiendifferenz der Funktionsflächen (5, 7) anschließt.

6. Künstliches Gelenk nach Anspruch 5,
**dadurch gekennzeichnet,** daß
der Endbereich (10) sich über einen Winkelbereich von ca. 70 ° erstreckt.

7. Künstliches Gelenk nach einem der Ansprüche 1 bis 6,
**dadurch gekennzeichnet,** daß

die Radiendifferenz im Abrollbereich (9) maximal 20 % des Radius der beiden Funktionsflächen (5, 7) im Auflagebereich (8) beträgt.

8. Künstliches Gelenk nach einem der Ansprüche 2 bis 7,
**dadurch gekennzeichnet,** daß
die Radiendifferenz der Funktionsflächen (6, 11) beidseitig der Berührungspunkte K maximal 30 % des Radius der Funktionsflächen (5, 7) im Auflagebereich (8) beträgt.

9. Künstliches Gelenk nach einem der Ansprüche 1 bis 8,
**dadurch gekennzeichnet,** daß
die Funktionsflächen (5, 7), (6, 11) eines der Gelenkteile (2, 3) sowohl in der frontalen Ebene als auch in der Querebene kreisförmig sind und denselben Radius besitzen, der dem Radius der Funktionsflächen (5, 7) im Auflagebereich (8) entspricht.

10. Künstliches Gelenk nach Anspruch 9,
**dadurch gekennzeichnet,** daß
der Gelenkteil mit den radiengleichen Funktionsflächen (5, 6) die Gelenkpfanne (2) ist.

11. Künstliches Gelenk nach Anspruch 9 oder 10,
**dadurch gekennzeichnet,** daß
der Radius der radiengleichen Funktionsflächen (5, 6) ca. 25 mm beträgt.

12. Künstliches Gelenk nach Anspruch 10 oder 11,
**dadurch gekennzeichnet,** daß
in der frontalen Ebene der Radius des Gelenkkopfes (3) ausgehend vom Auflagebereich (8) sich von ca. 25 mm auf ca. 21 mm am Ende des Abrollbereichs (9) verringert.

13. Künstliches Gelenk nach einem der Ansprüche 10 bis 12,
**dadurch gekennzeichnet,** daß
in der Querebene gesehen der Radius des Gelenkkopfes (3) ca. 12 % kleiner ist als der Radius der Funktionsflächen (5, 7) im Auflagebereich (8), insbesondere 22 mm.

14. Künstliches Gelenk nach Anspruch 13,
**dadurch gekennzeichnet,** daß
der Radius $R_{K2}$ des Gelenkkopfes (3) ausgehend von seinem maximalen Wert in der Querebene gesehen sich in dorsaler Richtung um ca. 3 mm über ein Umfangswinkel von 90 ° und in ventraler Richtung über ein Umfangswinkel von 90 ° um maximal 1 mm verringert.

**Claims**

1. An endoprosthesis for the human shoulder joint, comprising two joint parts, a joint ball and a joint socket, which move with respect to one another and have spherical joint surfaces, the curvature ratios of their functional surfaces being convex-concave both in the frontal plane and in a transverse plane perpendicular thereto, and the joint geometry of the functional surfaces with respect to one another being determined in each of the two planes by a dimeric joint chain, that is to say a joint chain having two joint axes, which runs through the rotational centres of the functional surfaces of the respective sectional contours, characterized in that the joint ball (3) and the joint socket (2) are constructed in respect of their functional surfaces such that, in their position in the hanging condition of the arm, which corresponds to the rest position of the arm, the functional surfaces (5, 7) on both sides are in linear contact with one another in the frontal plane and have the same radius of curvature in the support region (8), and adjoining this there is a rolling region (9) in which the radii $R_{P1}$, $R_{K1}$ of the two functional surfaces (5, 7) are selected such that a widening gap portion extends in the peripheral direction between the two joint parts (2, 3).

2. An artificial joint according to Claim 1, characterized in that, in the transverse plane which runs in the radial direction through the respective contact point K of the functional surfaces (6, 11) of the joint parts (2, 3), the radii $R_{P2}$, $R_{K2}$ of the functional surfaces (6, 11) are dimensioned such that a peripherally widening gap is produced in each case on either side of the contact point K.

3. An artificial joint according to Claim 1 or 2, characterized in that the rolling region (9) extends over a peripheral region of the joint ball (3) of approximately 180 °.

4. An artificial joint according to one of Claims 1 to 3, characterized in that the support region (8) extends over a peripheral region of the joint ball (3) of approximately 20 °.

5. An artificial joint according to one of Claims 1 to 4, characterized in that an end region (10) having a constant radial difference of the functional surfaces (5, 7) adjoins the rolling region (9) in the peripheral direction.

6. An artificial joint according to Claim 5, characterized in that the end region (10) extends over an angle range of approximately 70 °.

7. An artificial joint according to one of Claims 1 to 6, characterized in that the radial difference in the rolling region (9) is a maximum of 20 % of the radius of the two functional surfaces (5, 7) in the support region (8).

8. An artificial joint according to one of Claims 2 to 7, characterized in that the radial difference of the functional surfaces (6, 11) on either side of the contact points K is a maximum of 30 % of the radius of the functional surfaces (5, 7) in the support region (8).

9. An artificial joint according to one of Claims 1 to 8, characterized in that the functional surfaces (5, 7), (6, 11) of one of the joint parts (2, 3) are circular both in the frontal plane and in the transverse plane and have the same radius, which corresponds to the radius of the functional surfaces (5, 7) in the support region (8).

10. An artificial joint according to Claim 9, characterized in that the joint part having the functional surfaces (5, 6) of the same radius is the joint socket (2).

11. An artificial joint according to Claim 9 or 10, characterized in that the radius of the functional surfaces (5, 6) of the same radius is approximately 25 mm.

12. An artificial joint according to Claim 10 or 11, characterized in that, in the frontal plane, the radius of the joint ball (3) is reduced from approximately 25 mm, starting from the support region (8), to approximately 21 mm at the end of the rolling region (9).

13. An artificial joint according to one of Claims 10 to 12, characterized in that, as seen in the transverse plane, the radius of the joint ball (3) is approximately 12 % smaller than the radius of the functional surfaces (5, 7) in the support region (8), and is in particular 22 mm.

14. An artificial joint according to Claim 13, characterized in that, starting from its maximum value, the radius $R_{K2}$ of the joint ball (3) is reduced - as seen in the transverse plane - by approximately 3 mm over a peripheral angle of 90 ° in the dorsal direction and by a maximum of 1 mm over a peripheral angle of 90 ° in the ventral direction.

**Revendications**

1. Endoprothèse pour l'articulation de l'épaule humaine, comportant deux éléments d'articulation mobiles entre eux : une tête d'articulation, et une cavité articulaire à surfaces sphériques, la relation de courbure de leurs surfaces fonctionnelles étant convexe-concave, aussi bien dans le plan frontal que dans un plan transversal perpendiculaire à celui-ci, la géométrie d'articulation des surfaces fonctionnelles entre elles dans chacun des deux plans étant déterminée par une chaîne d'articulation dimère, c'est-à-dire une chaîne d'articulation à deux axes d'articulation qui passe par les centres de rotation des surfaces fonctionnelles des contours de coupe respectifs,

caractérisée en ce que les surfaces fonctionnelles de la tête d'articulation (3) et de la cavité articulaire (2) sont agencées de manière à ce que, dans la position correspondant à la position de repos du bras, lorsque celui-ci pend, les surfaces fonctionnelles (5, 7) de part et d'autre soient en contact linéaire dans le plan frontal, et présentent le même rayon de courbure dans la région de contact (8), cette région étant suivie d'une région de déroulement (9) où les rayons $R_{P1}$, $R_{K1}$ des deux surfaces fonctionnelles (5, 7) sont choisis de manière à ce que, dans la direction circonférentielle, une région de fente qui s'élargit s'étende entre les deux éléments d'articulation (2, 3).

2. Articulation artificielle selon la revendication 1, caractérisée en ce que, dans le plan transversal qui passe dans la direction radiale par le point de contact K respectif des surfaces fonctionnelles (6, 11) des éléments d'articulation (2, 3), les rayons $R_{P2}$, $R_{K2}$ des surfaces fonctionnelles (6, 11) sont prévues de manière à obtenir de part et d'autre du point de contact K une fente qui s'élargit dans la direction périphérique.

3. Articulation artificielle selon la revendication 1 ou 2, caractérisée en ce que la région de déroulement (9) s'étend sur une région périphérique de la tête d'articulation (3) de 180° environ.

4. Articulation artificielle selon l'une des revendications 1 à 3, caractérisée en ce que la région de contact (8) s'étend sur une région périphérique de la tête d'articulation (3) de 20° environ.

5. Articulation artificielle selon l'une des revendications 1 à 4, caractérisée en ce que la région de déroulement (9) est suivie dans la direction périphérique d'une région terminale (10) à différence de rayons constante des surfaces fonctionnelles (5, 7).

6. Articulation artificielle selon la revendication 5, caractérisée en ce que la région terminale (10) s'étend sur une région angulaire de 70° environ.

7. Articulation artificielle selon l'une des revendications 1 à 6, caractérisée en ce que la différence de rayons dans la région de déroulement (9) est de 20% maximum du rayon des deux surfaces fonctionnelles (5, 7) dans la région de contact (8).

8. Articulation artificielle selon l'une des revendications 2 à 7, caractérisée en ce que la différence de rayons des surfaces fonctionnelles (6, 11) de part et d'autre des points de contact K est de 30% maximum du rayon des surfaces fonctionnelles (5, 7) dans la région de contact (8).

9. Articulation artificielle selon l'une des revendications 1 à 8, caractérisée en ce que les surfaces fonctionnelles (5, 7) (6, 11) de l'un des éléments d'articulation (2, 3) sont circulaires aussi bien dans le plan frontal que dans le plan transversal, et présentent le même rayon qui correspond au rayon des surfaces fonctionnelles (5, 7) dans la région de contact (8).

10. Articulation artificielle selon la revendication 9, caractérisée en ce que l'élément d'articulation dont les surfaces fonctionnelles (5, 6) ont le même rayon est la cavité articulaire (2).

11. Articulation artificielle selon la revendication 9 ou 10, caractérisée en ce que le rayon des surfaces fonctionnelles (5, 6) à rayons identiques est de 25 mm environ.

12. Articulation artificielle selon la revendication 10 ou 11, caractérisée en ce que, dans le plan frontal, le rayon de la tête d'aritculation (3) diminue, en partant de la région de contact (8) ou sa longueur est de 25 mm environ, pour atteindre 21 mm environ à la fin de la région de déroulement (9).

13. Articulation artificielle selon l'une des revendications 10 à 12, caractérisée en ce que, vu dans le plan transversal, le rayon de la tête d'articulation (3) est inférieur de 12% environ au rayon des surfaces fonctionnelles (5, 7) dans la région de contact (8), en particulier de 22 mm.

14. Articulation artificielle selon la revendication 13, caractérisée en ce que le rayon $R_{K2}$ de la tête d'articulation (3) diminue dans la direction dorsale de 3 mm environ en partant de sa valeur maximum, sur un angle inscrit de 90°, et dans la direction ventrale, de 1 mm maximum sur un angle inscrit de 90°.

FIG.1

FIG.2

FIG.3